# EUROPÄISCHE PATENTSCHRIFT

(11) **EP 3 040 059 B1**
(45) Veröffentlichungstag und Bekanntmachung des Hinweises auf die Patenterteilung: **18.07.2018**
(21) Anmeldenummer: 15199723.6
(22) Anmeldetag: 14.12.2015
(51) Int. Cl.: A61F 5/01, A61H 1/02, A61H 9/00

(54) **EINRICHTUNG ZUR BEHANDLUNG VON GELENKSEINSTEIFUNGEN DER HAND**
DEVICE FOR THE TREATMENT OF JOINT REINFORCEMENTS FOR THE HAND
DISPOSITIF DE TRAITEMENT DES RAIDISSEMENTS ARTICULAIRES DE LA MAIN

(30) Priorität: 30.12.2014 DE 102014119715
(43) Veröffentlichungstag der Anmeldung: 06.07.2016
(73) Patentinhaber: Kemper, Heiner, 59073 Hamm (DE)
(72) Erfinder: Kemper, Heiner, 59073 Hamm (DE)
(74) Vertreter: Limbeck, Achim

(56) Entgegenhaltungen:
- AT-B- 404 427
- US-A- 3 937 215
- US-A- 5 020 515
- US-A- 5 437 620

## Beschreibung

### Technisches Gebiet

Die vorliegende Erfindung betrifft eine Einrichtung zur Behandlung von Gelenkseinsteifungen der Hand zur prae- und postoperativen Anwendung sowie zur Kräftigung der Finger-, Hand- und Unterarmmuskulatur, wobei die Einrichtung ein flexibles, pneumatisch beaufschlagbares Formteil zur Dehnung der Hand umfasst, welches innerhalb der Handinnenfläche mittels einer Handpumpe oder einer elektrischen Pumpe aufgeblasen wird. Die vorliegende Erfindung findet insbesondere In der Handchirurgie, der Ergotherapie sowie in der Physiotherapie Anwendung.

### Stand der Technik

Fingerorthesen sind aus dem Stand der Technik in unterschiedlichsten Ausführungsformen als orthopädische Hilfs- und Heilmittel unlängst bekannt. Diese sind dazu geeignet, einem Gelenk Halt zu geben, ein Überstrecken eines Gelenks zu korrigieren sowie schmerzhafte Fehlstellungen zu verhindern. Diesbezüglich seien die Druckschriften US 4,270,538 sowie die EP 1813233 A1 beispielhaft erwähnt. Bei den aus dem Stand der Technik bekannten Orthesen handelt es sich stets um starre Schienen, die bspw. mittels eines vorspringenden Zugmittels betroffene Finger an kleinen Seilen nach oben ziehen. Während des Schlafens wirken diese störend.

Bisher übliche Schienen verlaufen über das Handgelenk und den Handrücken und steifen so bei länger angelegter Schiene das Handgelenk ein, was wiederum langwierige Ergo- oder Physiotherapiesitzungen nach sich zieht. Ferner werden die bekannten Orthesen in der Handinnenfläche stabilisiert. Bei operativen Verfahren durch Folgeschäden nach Verletzungen der Handwurzel sowie bei operativen Verfahren bei Morbus Dupuytren und der komplexen Therapie des Morbus Sudeck (CRPS) sind diese Orthesen völlig ungeeignet, da diese unter anderem auf die Wundnarben der Mittelhand drücken.

Nach einer Morbus Sudeck Erkrankung ist es nach einer wochen- oder sogar monatelangen Schonhaltung der Hand nicht mehr möglich, diese zu öffnen. Das gleiche gilt für die Morbus Dupytren Erkrankung.

Aus der US 5,383,827 A ist ein aufblasbares Handgerät zur Unterstützung der therapeutischen Ausübung der Finger und des Daumens der Hand eines Patienten bekannt, wobei die Vorrichtung umfasst: eine aufblasbare Blase zum Positionieren zwischen der Handfläche und den Fingern der Hand eines Patienten. Eine Seite der Blase ist der Handfläche angepasst und eine andere Seite liegt im Wesentlichen gegenüber der Handflächenseite auf. Weiterhin ist eine äußere Naht um den Umfang der Blase zur Bildung einer Innenkammer vorgesehen, welche zum Aufnehmen und Halten eines Aufblasfluids geeignet ist. Ferner umfasst das Handgerät eine Einrichtung zum Befestigen der aufblasbaren Blase in der Hand des Patienten, wobei eine nicht aufgeblasene Blase unter den Fingern platziert wird und dann aufgepumpt wird, um die Finger zu erstrecken und eine Abduktion zu erreichen. Die in der US 5,383,827 A beschriebene Einrichtung ist dadurch gekennzeichnet ist, dass sie ebenfalls ein flexibles, pneumatisch beaufschlagbares Formteil (Blase) umfasst, welches derart ausgeformt und in der Handinnenfläche anlegbar ist, dass es zwischen den Fingern sowie dem Hand- und/oder Daumenballen anliegt. Nachteilig hierbei ist allerdings, dass in der US 5,383,827 A zwischen dem Formteil und der Mittelhand kein Hohlraum entsteht, so dass die Nutzung des Handgerätes für den Patienten äußerst schmerzhaft ist.

Aus der US 5152740 A ist ebenfalls ein pneumatisch betätigbares Luftpolster für die Handinnenfläche beschrieben, welche allerdings ebenfalls nicht mit einer einen Hohlraum bildenden Ausführung beschrieben ist.

In der US 5437620 A die den nächstliegenden Stand der Technik darstellt, wird ein Handgerät beschrieben, welches zwar zwei Zonen umfasst, mittels deren Hilfe die Hand gespreizt werden kann, allerdings erfolgt der Spreizvorgang hier nicht mittels Aufpumpen der beiden an den Handinnenflächen vorhandenen Luftpolster, sondern vielmehr durch Verschieben eines der Polster an einer Führungsschiene. Dies hat den wesentlichen Nachteil, dass durch das Verschieben der Schiene lediglich eine lineare und damit entgegen der Anatomie vorgesehene Ausdehnung des zweiten Polsters und mithin der Hand erreicht wird, was ebenfalls Schmerzen verursacht.

In der 1098165 B ist ein Handgerät zur Übung der Muskulatur und des Gefäß- und Nerven-Systems bekannt, welches aus zwei unter Spannung und unter Änderung ihres Winkels gegeneinanderdrückbaren, miteinander verbundenen Platten besteht, wobei in den spitzwinklig divergierenden, gegeneinanderfedernden ebenen Griffplatten eine oder mehrere Ausnehmungen vorgesehen sind. Die Nutzung dieses Handgeräts zur Behandlung von Gelenkseinsteifungen der Hand ist bereits deshalb nicht möglich, da es sich nicht zwischen den geschlossenen Handinnenfläche platzieren lässt, darüber hinaus würde es bei der Anwendung bereits konstruktionsbedingt zu starken Schmerzen führen.

### Darstellung der Erfindung

Der vorliegenden Erfindung liegt die Aufgabe zu Grunde, eine Einrichtung zu schaffen, welche die vorgenannten Nachteile ausräumt und welche geeignet ist, die Hand, die in einer Pfötchenstellung oder halb geschlossenen Faust verharrt, wieder zu öffnen, ohne dabei mögliche Wunden in der Handinnenfläche zu berühren. Zugleich sollten alle Gelenke frei liegen, um an ergotherapeutischen Maßnahmen während des Tragens der Einrichtung teilnehmen zu können.

Erfindungsgemäß wird die voranstehende Aufgabe gemäß dem Oberbegriff des Anspruchs 1 in Verbindung mit den kennzeichnenden Merkmalen gelöst. Vorteilhafte Ausgestaltungen und Weiterbildungen der erfindungsgemäßen Einrichtung sind in den abhängigen Unteransprüchen angegeben.

Erfindungsgemäß ist eine Einrichtung der eingangs genannten Art dadurch gekennzeichnet, dass die Luftpolster mittels einer an dem Formteil vorhandenen Unterteilung, Verklebung, Verschweißung, Naht oder ein dergleichen geeignetes Mittels zwischen den Luftpolstern gebildet werden, wobei die Luftpolster durch mindestens zwei an dem Formteil vorgesehene Luftkammern gebildet werden.

Der Vorteil der erfindungsgemäßen Einrichtung besteht in erster Linie darin, dass alle Hand- Finger und Knöchelgelenke freiliegen. Ein Einsteifen der Gelenke bei längerer Anwendung ist somit ausgeschlossen. Durch die frei einstellbare Druckintensität des Formteils sind während der gesamten Tragezeit auch Greif- und Kraftübungen der Hand möglich. Der Patient kann die Einrichtung je nach Schmerzempfindlichkeit individuell einstellen. Dadurch wird eine schonende und weitestgehend schmerzfreie Dehnung der Hand erreicht. Die Einrichtung kann - ohne störend zu wirken - über Nacht getragen werden. Hierdurch werden ergotherapeutische Sitzungen sinnvoll unterstützt, gerade wenn die Einrichtung die ganze Zeit getragen wird. Die Einrichtung berührt keine Wunden in der Mittelhandfläche. Sie braucht nicht durch einen Arzt angepasst werden und passt sich automatisch den Gegebenheiten an. Zum Duschen und Baden wird sie einfach abgenommen und danach wieder angelegt.

### Kurzbeschreibung der Zeichnungen

Weitere Ziele, Merkmale, Vorteile und Anwendungsmöglichkeiten der erfindungsgemäßen Einrichtung ergeben sich aus der nachfolgenden Beschreibung eines Ausführungsbeispiels anhand der Zeichnung. Dabei bilden alle beschriebenen und/oder bildlich dargestellten Merkmale für sich oder in beliebiger Kombination den Gegenstand der Erfindung, unabhängig von der Zusammenfassung in einzelnen Ansprüchen oder deren Rückbeziehung.

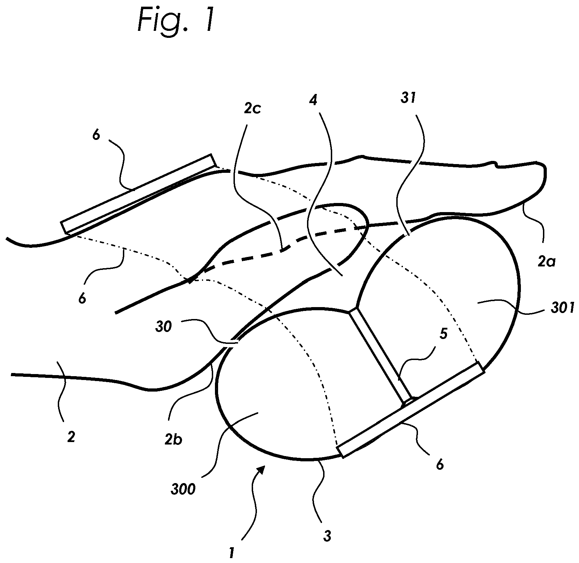

In der Zeichnung zeigt
Fig.1 die erfindungsgemäße Einrichtung an einer Hand.

### Ausführung der Erfindung

Wie aus Fig.1 ersichtlich, weist das mindestens eine Formteil 3 mindestens zwei zonenartig verteilte Luftpolster 30,31 auf, welche ihrerseits derart ausgeformt und an Fingern 2a und Hand- sowie Daumenballen 2b der Handinnenfläche derart anlegbar sind, dass sie ausschließlich an den Fingern 2a sowie dem Hand- und/oder Daumenballen 2b anliegen.

Das Formteil 3 bzw. seine Luftpolster 30,31 werden vorzugsweise mittels einer Handpumpe oder einer elektrischen Pumpe aufgeblasen, wobei vorteilhaft ein Druckmesser zur Anzeige des Innendrucks in dem Formteil 3 vorgesehen ist. Das Formteil 3 ist in ihrem Volumen somit in dieser besonders vorteilhaften Ausführung stufenlos einstellbar und kann mit mehr oder weniger Luft gefüllt werden kann, wobei die Luft in dem Formteil auch jegliches Gas sein kann.

Die Luftpolster 30,31 werden vorzugsweise mittels einer an dem Formteil 3 vorhandenen Unterteilung, Verklebung, Verschweißung, Naht oder ein dergleichen geeignetes Mittels zwischen den Luftpolstern 30,31 gebildet. Ganz besonders bevorzugt wird die Unterteilung zwischen den Luftpolstern 30,31 durch mindestens einen das Formteil 3 umfassenden Bügel oder eine Klammer 5 gebildet.

Die Luftpolster 30,31 werden durch mindestens zwei an dem Formteil (3) vorgesehene Luftkammern (300,301) gebildet.

Ganz besonders bevorzugt umfasst die Einrichtung 1 - wie in Fig.1 dargestellt - eine Manschette 6, welche um die gesamte Hand 2 gelegt und an dieser befestigt werden kann, wobei die Manschette 6 zumindest bereichsweise als Formteil 3 ausgebildet oder an welcher bereichsweise mindestens ein Formteil 3 angeordnet ist.

Die erfindungsgemäße Einrichtung 1 beschränkt sich in ihrer Ausführung nicht auf die vorstehend angegebenen bevorzugten Ausführungsformen. Vielmehr sind eine Vielzahl von Ausgestaltungsvariationen denkbar, welche von der dargestellten Lösung auch bei grundsätzlich anders gearteter Ausführung Gebrauch machen.

### Liste der Bezugsziffern

- 1: Einrichtung
- 2: Hand
- 2a: Finger
- 2b: Hand- / Daumenballen
- 2c: Mittelhand
- 3: Formteil
- 4: Hohlraum
- 5: Klammer
- 6: Manschette
- 30,31: Luftpolster
- 300,301: Luftkammern

## Patentansprüche

1. Einrichtung (1) zur Behandlung von Gelenkseinsteifungen der Hand (2) zur prae- und postoperativen Anwendung sowie zur Kräftigung der Finger-, Hand- und Unterarmmuskulatur, wobei die Einrichtung (1) ein flexibles, pneumatisch beaufschlagbares Formteil (3) zur Dehnung der Hand umfasst, welches innerhalb der Handinnenfläche mittels einer Handpumpe oder einer elektrischen Pumpe aufgeblasen wird, wobei das Formteil (3) mindestens zwei zonenartig verteilte Luftpolster (30,31) aufweist, die derart ausgeformt und in der Handinnenfläche anlegbar sind, dass das Formteil (3) ausschließlich an den Fingern (2a) sowie dem Hand- und/oder Daumenballen (2b) anliegt, wobei zwischen dem mindestens einen Formteil und der Mittelhand (2c) ein Hohlraum (4) entsteht,
**dadurch gekennzeichnet, dass**
die Luftpolster (30,31) mittels einer an dem Formteil (3) vorhandenen Unterteilung, Verklebung, Verschweißung, Naht oder eines dergleichen geeigneten Mittels zwischen den Luftpolstern (30,31) gebildet werden, wobei die Luftpolster (30,31) durch mindestens zwei an dem Formteil (3) vorgesehene Luftkammern (300,301) gebildet werden.

2. Einrichtung (1) nach Anspruch 1,
**dadurch gekennzeichnet, dass**
die Unterteilung zwischen den Luftpolstern (30,31) durch mindestens einen das Formteil (3) umfassenden Bügel oder eine Klammer (5) gebildet wird.

3. Einrichtung (1) nach den Ansprüchen 1 und 2,
**dadurch gekennzeichnet, dass**
das Formteil (3) in ihrem Volumen stufenlos einstellbar ist.

4. Einrichtung (1) nach einem der vorangegangenen Ansprüche,
**dadurch gekennzeichnet, dass**
das Formteil (3) mit mehr oder weniger Luft gefüllt werden kann.

5. Einrichtung (1) nach einem der vorangegangenen Ansprüche,
**dadurch gekennzeichnet, dass**
die Einrichtung (1) eine Manschette (6) umfasst, welche um die gesamte Hand (2) gelegt und an dieser befestigt werden kann, wobei die Manschette (6) zumindest bereichsweise als Formteil (3) ausgebildet oder an welcher bereichsweise mindestens ein Formteil (3) angeordnet ist.

## Claims

1. Device (1) for the treatment of joint stiffening or ankylosis of the hand (2), for pre- and post-surgical application and for the reinforcement of finger, hand and forearm muscles, whereby the device (1) is a flexible, pneumatically pressurizable form part (3) for stretching the hand, which is pumped up by means of a manual or an electric air pump when the form part (3) is in place within the palm of the hand, whereby the form part (3) comprises at least two air cushion arranged in zones (30, 31), which are formed and located in the palm of the hand, so that the form part (3) is in contact exclusively with the fingers (2a) as well as the ball of the hand or thumb or thenar eminence (2b), whereby a hollow (4) is created between one of the form parts and the middle hand (2) is created;
**characterized in that**
the air cushions (30, 31) are formed by means of division, gluing, welding, seam or similar means between the air cushions arranged centrally on the form part (3), whereby the air cushions (30, 31) are formed by at least two air chambers 300, 301) provided on the form part (3).

2. Device (1) according to Claim 1,
**characterized in that**
the division between the air cushions (30, 31) is formed by at least one bracket or clamp (5) encompassing the form part (3).

3. Device (1) according to Claims 1 and 2,
the volume of the form part (3) is continuously adjustable.

4. Device (1) according to any of the preceding Claims,
**characterized in that**
the form part (3) can be filled with more or less air.

5. Device (1) according to any of the preceding Claims,
**characterized in that**
the device (1) comprises a cuff (6) which is placed around and fasten to the entire hand (2), whereby the cuff (6) is formed, at least in sections, as a form part (3), or on which, at least in sections, a form part (3) is arranged.

## Revendications

1. Dispositif (1) de traitement des raidissements articulaires de la main (2) pour une utilisation pré- et post-opératoire, ainsi que pour le renforcement des muscles du doigt, de la main et de l'avant-bras, tout en notant que ledit dispositif (1) comprend une pièce moulée (3) souple et pneumatiquement agencée pour étirer la main, qui au moyen d'une pompe manuelle ou électrique, est gonflée dans le creux de la paume de main, à l'intérieur duquel la pièce moulée (3) présente au moins deux coussins d'air (30,31) disposés par zone, ceux-ci étant formés et pouvant être appliqués dans la paume de la main, de telle sorte que la pièce moulée (3) repose exclusivement sur les doigts (2a) ainsi que sur la main et/ou sur la région du pouce (2b), créant ainsi un espace vide (4) qui se forme au moins entre la pièce moulée et le métacarpe (2c).
**Caractérisé par le fait que**
La pièce moulée (3) dispose au moins de deux coussins d'air disposés par zone (30,31), qui sont formés de telle manière qu'ils peuvent être appliqués dans le creux de la paume de main si bien que la pièce moulée (3) repose uniquement sur les doigts (2a) ainsi que sur la main et/ou sur la région du pouce (2b), créant ainsi un espace vide (4) qui se forme au moins entre la pièce moulée et le métacarpe (2c), lesdits coussins d'air (30,31) sont formés au niveau de la pièce moulée (3) sur la subdivision de moulage, collage, soudure, couture ou tout autre moyen approprié existant entre les coussins d'air (30,31) tout en notant qu'au moins deux chambres à air (300,301) sont prévues sur la pièce moulée (3).

2. Dispositif (1) selon la revendication1,
**caractérisé par le fait que**
la subdivision entre les coussins d'air (30,31) est formée à l'aide d'au moins un étrier ou clip (5) qui entoure la pièce moulée (3).

3. Dispositif (1) selon les revendications 1 et 2,
**caractérisé par le fait que**
la pièce moulée (3) étant adaptée au volume, est réglable en continu.

4. Dispositif (1) selon l'une des revendications précédentes,
**Caractérisé par le fait que**
la pièce moulée (3) peut être remplie avec plus ou moins d'air.

5. Dispositif (1) selon l'une des revendications précédentes,
**Caractérisé par le fait que**
le dispositif (1) comprend un manchon (6) qui peut être placé autour de toute la main (2) et fixé à celle-ci, le manchon (6) étant au moins partiellement formé comme une pièce moulée (3) ou disposé au moins partiellement en pièce moulée (3).
